(19) 

**Europäisches Patentamt**
**European Patent Office**
**Office européen des brevets**

(11) **EP 2 343 381 A1**

(12) **EUROPEAN PATENT APPLICATION**

(43) Date of publication:
**13.07.2011 Bulletin 2011/28**

(51) Int Cl.:
*C12Q 1/04* (2006.01)        *C12Q 1/66* (2006.01)
*C12M 1/34* (2006.01)

(21) Application number: **11150476.7**

(22) Date of filing: **10.01.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **24.12.2010 JP 2010287527**
**08.01.2010 JP 2010002726**

(71) Applicant: **Hitachi Plant Technologies, Ltd.**
**Tokyo (JP)**

(72) Inventor: **Yamamoto, Harumasa**
**Tokyo 170-8466 (JP)**

(74) Representative: **Beetz & Partner**
**Patentanwälte**
**Steinsdorfstrasse 10**
**80538 München (DE)**

(54) **Inspection method of airbone floating bacteria and its apparatus**

(57)    To present an inspection method of airborne floating bacteria and its apparatus capable of determining the quantity of ATP contained in an inspection sample by measuring the light emission quantity by a biological light emission reaction, and counting the number of cells in the airborne floating bacteria, while evaluating the reliability of the ATP light emission reagent to be used and the inspection results at the same time.

In an inspection method of airborne floating bacteria for performing a biological light emission reaction by using an ATP light emission reagent on the basis of the ATP derived from viable cells in captured airborne floating bacteria in the air contained in the inspection sample, measuring the light emission quantity due to the biological light emission reaction, determining the ATP quantity contained in the inspection sample, and counting the number of cells in the airborne floating bacteria, the light emission quantity of the ATP light emission reagent is measured, and a measured value of light emission quantity of the ATP light emission reagent and a predetermined theoretical value corresponding to the measured value are compared, and thereby the reliability of the ATP light emission reagent to be used and the inspection results is evaluated at the same time.

FIG. 3

```
Start of measurement
        │
Capturing of bacteria
        │
Recovery of bacteria
        │
ATP elimination
        │
ATP extraction
        │
ATP measurement
        │
Judging of results
        │
Display of results
        │
       END
```

**Description**

**Technical Field**

The present invention relates to an inspection method of airborne floating bacteria and its apparatus, and more particularly to an inspection method of airborne floating bacteria and its apparatus for performing a biological light emission reaction by using an ATP light emission reagent on the basis of adenosine triphosphate (ATP) derived from viable cells in captured airborne floating bacteria, measuring the light emission quantity due to the biological light emission reaction, determining the ATP quantity contained in the inspection sample, and counting the number of cells in the airborne floating bacteria, together with the reliability evaluation of the ATP light emission reagent to be used and the inspection results.

**Background Art**

Conventionally, as the inspection method of airborne floating bacteria, for example, the present applicant formerly proposed a capturing device of airborne floating bacteria and an analysis method by using the same, in which a biological light emission reaction is carried out by using an ATP light emission reagent, the light emission quantity due to the biological light emission reaction is measured, and the ATP quantity contained in the inspection sample is determined, and thereby the number of cells contained in the airborne floating bacteria is counted (see, for example, patent literature 1).

This inspection method of airborne floating bacteria includes a capturing process of capturing airborne floating bacteria by operating a capturing apparatus, by using the capturing apparatus provided with an inhalation nozzle, having a container, and a capturing device composed of a capturing carrier containing a high polymer of phase transfer between gel and sol at a temperature contained in the container at 40°C or less, or more specifically in a range of 15°C or more to 35°C or less, a bacteria recovery process of transferring the phase of the capturing carrier from gel state to sol state, and collecting a bacterial suspension solution (sample), an ATP eliminating process of eliminating extracellular ATP and ATP in dead cells in the sample, a filtering process of filtering liquid components including debris smaller than the bacterial cells, and capturing the viable cells in the sample on a filter, an ATP extracting process of dissolving cell walls of the viable cells, and extracting the ATP contained in the cytoplasm of the viable cells in the sample solution, an ATP recovery process of obtaining the sample solution containing the ATP extracted from the viable cells, and an ATP measuring process of determining the ATP content in the sample, and presenting the inspection result as the number of airborne floating cells per unit volume, in which the ATP measuring process includes the following steps.

First, prior to the ATP measuring process, it is a step of measuring the light emission quantity of the baseline as the background noise of the measuring system.

In the light emission measurement, a photomultiplier tube and a comparator are combined, and by using the photomultiplier tube of photon counting system for issuing one pulse per one photon of the incident light entering the photomultiplier tube, the quantity of light of light emission is counted as the number of generated pulses (in the unit of cps or counts per second).

This is determined by measuring the light emission quantity of the ATP light emission reagent for a predetermined period before injection of the ATP, and the obtained average is recorded as C1.

In succession, in a predetermined period immediately after injection of a specified amount of ATP sample adjusted to a known ATP content (hereinafter called a standard ATP sample), the light emission quantity of the ATP light emission reagent is measured, and the first peak value is obtained as C2, and by subtracting the value of C1, the effective light emission quantity of the standard ATP sample is determined, and this effective light emission quantity is divided by the concentration K1 and the dispensing amount V1 of the ATP in the standard ATP sample, thereby determining the light emission coefficient R1 per unit ATP as the measurement sensitivity (formula 1).

Next, in a certain time after measurement of C2, the light emission quantity of the ATP light emission reagent in a predetermined period is measured and recorded as average C3, and this is followed by a step of measuring the light emission quantity of the ATP light emission reagent in a predetermined period right after injection of ATP sample derived from the viable cells in the airborne floating bacteria captured in the air of the sample solution (hereinafter called "captured ATP sample"), obtaining the first peak value as C4, subtracting the value of C3, and determining the effective light emission quantity of the captured ATP sample.

Further, it is multiplied by the light emission coefficient R1 per unit ATP which is the measurement sensitivity obtained beforehand, and it is further divided by the dispensing amount V2 of the captured ATP sample, and thereby the conversion amount S of the ATP of the captured ATP sample is determined (formula 2).

Moreover, the conversion amount S of the ATP of the captured ATP sample is divided by the average of the ATP content per one viable cell (about 2 amol), and the average number of viable cells in the captured ATP sample is obtained, and the average number of viable cells in the captured ATP sample is divided by the volume of the gas sample, so that the average number of viable cells contained in the gas sample of unit volume can be determined.

$$R1 = (C2-C1) / (K1 \cdot V1) \qquad \text{(formula 1)}$$

R1: light emission coefficient per unit ATP (cps/(amol.μL)).
C1: average value of light emission quantity of ATP light emission reagent in a predetermined period before injection of ATP (cps).
C2: first peak value of measured values of light emission quantity of ATP light emission reagent in a predetermined period right after injection of specified amount of ATP (cps).
K1: concentration of specified amount of ATP (amol).
V1: dispensing amount of specified amount of ATP (μL).

$$S = (C4-C3) \cdot R1/V2 \text{ (amol)} \qquad \text{(formula 2)}$$

S: conversion amount of ATP derived from viable cells in airborne floating bacteria captured in the air (amol).
C3: average value of light emission quantity of ATP light emission reagent in a predetermined period after lapse of specific time after injection specified amount of ATP (cps).
C4: first peak value of measured values of light emission quantity of ATP light emission reagent in a predetermined period right after injection of ATP derived from viable cells in airborne floating bacteria captured in the air (cps).
V2: dispensing amount of ATP derived from viable cells in airborne floating bacteria captured in the air (μL).

**Prior art literature**

**Patent literature**

[Patent literature 1] Japanese Patent Application Laid-Open No. 2009-139115

**Summary of the Invention**

**Problems to be Solved by the Invention**

In the conventional inspection method of airborne floating bacteria, the number of cells in the airborne floating bacteria can be counted by measuring the light emission quantity of the ATP light emission reagent, and determining the ATP conversion amount, but the airborne floating bacteria captured in the capturing process of airborne floating bacteria are measured by an automatic measuring apparatus in a series of mechanized process from the subsequent bacteria recovery process to the ATP measurement process for measuring the light emission quantity of the ATP light emission reagent.
However, owing to disturbance factors, such as deterioration of dispensing and dividing precision of reagents due to adjustment failure of mechanical system of measuring apparatus, deterioration of performance of photomultiplier tube (PMT) as measuring means of light emission quantity, deterioration of reagents and other chemicals, non-uniform quality of reagents and other chemicals, and other external factors, the reliability of measured values of light emission quantity of the ATP light emission reagent is not evaluated sufficiently.
Accordingly, the final count values of bacterial cells and the measured values of light emission quantity of each ATP light emission reagent are indicated only as inspection results (as the average number of viable cells contained in the gas sample per unit volume) if abnormalities are present, and to judge whether the inspection values are abnormal or not, so far, we owe much to the experience of the inspection operator.
The present invention is intended to solve the problems of the conventional inspection method of airborne floating bacteria and its apparatus, and it is hence a primary object thereof to present an inspection method of airborne floating bacteria and its apparatus for determining the ATP quantity contained in the inspection sample by measuring the light emission quantity by biological light emission reaction, and counting the number of cells contained in the airborne floating bacteria, while evaluating the reliability of the ATP light emission reagent to be used and the inspection results.

**Means for Solving the Problems**

To achieve the object, a first aspect of the invention relates to an inspection method of airborne floating bacteria for performing a biological light emission reaction by using an ATP light emission reagent on the basis of the ATP derived from viable cells in captured airborne floating bacteria, measuring the light emission quantity due to the biological light emission reaction, determining the ATP quantity contained in the inspection sample, and counting the number of cells in the airborne floating bacteria, in which the ATP light emission reagent is evaluated by measuring the light emission quantity of the ATP light emission reagent about the following items (1) to (4), and measured values of light emission quantity of the ATP light emission reagent and predetermined theoretical values corresponding to the measures values are compared, and the reliability of the ATP light emission reagent to be used and the inspection results is evaluated at the same time. Said items (1) to (4) include:

(1) Light emission quantity of ATP light emission reagent in a predetermined period before injection of ATP.
(2) Light emission quantity of ATP light emission reagent in a predetermined period right after injection of specified amount of ATP.
(3) Light emission quantity of ATP light emission reagent in a predetermined period at the termination point of ATP light emission reaction by injecting a specified amount of ATP, and consuming the ATP by ATP light emission reaction after lapse of a predetermined time.
(4) Light emission quantity of ATP light emission reagent in a predetermined period right after injection of ATP derived from viable cells in airborne floating bacteria captured in the air.

In this case, if the variation coefficient of measured values of the light emission quantity of ATP light emission reagent of the above item (1) is more than 40%, it may be judged that the measured values are not reliable.

Or, if the balance of subtracting the average value of measured values of the light emission quantity of ATP light emission reagent of the above item (1) from the first peak value of measured values of the light emission quantity of ATP light emission reagent of the above item (2) is out of a range of -30% to +30% of the theoretical value of light emission quantity of a specified amount of ATP, it may be judged that the measured values are not reliable.

If the attenuation rate calculated by dividing the logarithmic difference of measured values at two points arbitrarily selected from measured values of the light emission quantity of ATP light emission reagent of the above item (2) by the time difference of the two points is out of a range of -0.2 dB/second to +0.2 dB/second of the theoretical value of attenuation rate of light emission quantity of a specified amount of ATP, it may be judged that the measured values are not reliable.

If the measured values of the light emission quantity of ATP light emission reagent of the above item (2) are out of a range of values of adding or subtracting the product of multiplying the value of the light emission quantity by the variation coefficient of measured values of the light emission quantity of ATP light emission reagent of the above item (1), to or from the value of the light emission quantity indicated by a virtual line having an inclination of the attenuation rate calculated by separating measured values of the light emission quantity of ATP light emission reagent of the above item (2) into two regions, a first half and a latter half, and dividing the average value of the two values of the average value of logarithm of each measured value of the first half and the average of logarithm of each measured value of the second half, by the time difference of the first half region and the second half region, it may be judged that the measured values are not reliable.

If the measured values of the light emission quantity of ATP light emission reagent of the above item (4) are out of a range of values of adding or subtracting the product of multiplying the value of the light emission quantity by the variation coefficient of measured values of the light emission quantity of ATP light emission reagent of the above item (1), to or from the value of the light emission quantity indicated by a virtual line having an inclination obtained as an inclination by multiplying the attenuation rate calculated by separating measured values of the light emission quantity of ATP light emission reagent of the above item (2) into two regions, a first half and a latter half, and dividing the average value of the two values of the average value of logarithm of each measured value of the first half and the average of logarithm of each measured value of the second half, by the time difference of the first half region and the second half region, and by adding the average of the light emission quantity of the ATP light emission reagent in the predetermined period, it may be judged that the measured values are not reliable.

If the first peak value of measured values of the light emission quantity of ATP light emission reagent of the above item (4) is more than 10 times of the limit for maintaining the linearity of light emission reaction of the first peak value of measured values of the light emission quantity of ATP light emission reagent of the above item (2), it may be judged that the inspection is disabled or that the measured values are not reliable.

If the variation coefficient of measured values of the light emission quantity of ATP light emission reagent of the above item (3) is out of a range of 0.5 times to 1.5 times of the variation coefficient of measured values of the light emission reagent of the above item (1), it may be judged that the measured values are not reliable.

If the first peak value of measured values of the light emission quantity of ATP light emission reagent of the above item (4) is smaller than the sum of adding the standard deviation of measured values of the light emission quantity of ATP light emission reagent of the above item (3) to the average value of measured values of the light emission quantity of ATP light emission reagent of the above item (3), it may be judged that the inspection is disabled or that the measured values are not reliable.

To achieve the same object, a second aspect of the invention relates to an inspection apparatus of airborne floating bacteria including airborne floating bacteria capturing means for capturing airborne floating bacteria in the air, inspection sample generating means for generating an inspection sample extracting an ATP derived from viable cells in the airborne floating bacteria captured by the airborne floating bacteria capturing means, light emission quantity measuring means of an ATP light emission reagent for performing a biological light emission reaction by using the ATP light emission reagent on the basis of the ATP derived from viable cells in the captured airborne floating bacteria captured in the air contained in the inspection sample, and measuring the light emission quantity due to the biological light emission reaction, and arithmetic means for determining the ATP quantity contained in the inspection sample on the basis of the measured light emission quantity of the ATP light emission reagent, and counting the number of cells in the airborne floating bacteria, further including reliability evaluating means for evaluating the reliability of the ATP light emission reagent and the inspection results, by comparing between measured values of light emission quantity of the ATP light emission reagent and predetermined theoretical values corresponding to the measures values, by measuring the light emission quantity of the ATP light emission reagent about the following items (1) to (4) about the ATP light emission reagent by the light emission quantity measuring means of the ATP light emission reagent.

(1) Light emission quantity of ATP light emission reagent in a predetermined period before injection of ATP.
(2) Light emission quantity of ATP light emission reagent in a predetermined period right after injection of specified amount of ATP.
(3) Light emission quantity of ATP light emission reagent in a predetermined period at the termination point of ATP light emission reaction by injecting a specified amount of ATP, and consuming the ATP by ATP light emission reaction after lapse of a predetermined time.
(4) Light emission quantity of ATP light emission reagent in a predetermined period right after injection of ATP derived from viable cells in airborne floating bacteria captured in the air.

**Effects of the Invention**

According to the inspection method of airborne floating bacteria in the first aspect of the invention, in the case of inspection of airborne floating bacteria by determining the ATP quantity contained in the inspection sample by measuring the light emission quantity due to biological light emission reaction, and counting the number of cells in the airborne floating bacteria, measured values of light emission quantity of the ATP light emission reagent to be measured and predetermined theoretical values corresponding to the measured values are compared, and thereby the reliability of the ATP light emission reagent to be used and the inspection results is evaluated, and the operation worker is informed of abnormality easily if there is any abnormality in the ATP light emission reagent to be used or in the inspection results, so that an excellent inspection method of airborne floating bacteria can be presented.

Heat noise of a photomultiplier tube used as light emission quantity measuring means for measuring the light emission quantity of an ATP light emission reagent is a finite value, and is nearly a constant value in a state free from external disturbance light, and the variation coefficient obtained by dividing the standard deviation of light emission quantity of ATP light emission reagent in a predetermined period before injection of ATP by the average number is 30 to 40% in a normal range in a combination of general photomultiplier, amplifier and comparator, and hence by judging that measured values are not reliable if the variation coefficient of measured values of the light emission quantity of ATP light emission reagent of the above item (1) is more than 40%, it is possible to check and remedy in an earlier stage in the event of deterioration or trouble of photomultiplier tube, abnormality of hardware of measurement, presence or absence of contamination of ATP light emission reagent by ATP, or deterioration of performance of ATP eliminating reagent, and thereby the reliability of the inspection results obtained by the inspection method may be maintained.

The first peak value of light emission quantity of the ATP light emission reagent in a predetermined period right after injection of standard ATP sample is the measured value of light emission quantity of the sample adjusted to a known ATP content, and if it is out of a range of -30% to +30% of the theoretical value in consideration of an error of 20% of the concentration of the standard ATP sample, and a variation of about 10% due to aging deterioration or reaction temperature of standard ATP sample, by judging that the measured values are not reliable, it is possible to check and remedy in an earlier stage in the event of deterioration or trouble of photomultiplier tube, abnormality of hardware of measurement, or checking of concentration of standard ATP sample, and thereby the reliability of the inspection results obtained by the inspection method may be maintained.

The light emission quantity of the ATP light emission reagent in a predetermined period right after injection of standard ATP begins to deteriorate gradually, and its attenuation rate is an intrinsic value (theoretical value) when the concentration and the amount of the standard ATP sample are constant, and if the attenuation rate calculated by dividing the logarithmic difference of measured values at two points (for example, beginning and end of a predetermined period) selected arbitrarily by the time difference of the two points is out of a range of -0.2 dB/second to +0.2 dB/second of the theoretical value, by judging that the measured values are not reliable, it is possible to check and remedy in an earlier stage in the event of abnormality of reaction conditions, for example, the temperature condition going out of a specified range, presence or absence of external disturbance light during measurement of light emission quantity, presence or absence of vibration during measurement of light emission quantity, and other troubles, and thereby the reliability of the inspection results obtained by the inspection method may be maintained.

The light emission quantity of the ATP light emission reagent in a predetermined period right after injection of standard ATP begins to deteriorate gradually and stably unless ATP is newly supplied after start of reaction, or the vibration or temperature changes suddenly, and the difference between the virtual line and measured values settles within a predetermined allowable range, and therefore if the measured values of the light emission quantity of ATP light emission reagent of the above item (2) are out of a range of values of adding or subtracting the product of multiplying the value of the light emission quantity by the variation coefficient of measured values of the light emission quantity of ATP light emission reagent of the above item (1), to or from the value of the light emission quantity indicated by the virtual line, by judging that the measured values are not reliable, it is possible to check and remedy in an earlier stage in the event of deterioration or trouble of photomultiplier tube, abnormality of reaction conditions, and other troubles, and thereby the reliability of the inspection results obtained by the inspection method may be maintained.

The light emission quantity of the ATP light emission reagent in a predetermined period right after injection of standard ATP begins to deteriorate gradually and stably unless ATP is newly supplied after start of reaction, or the vibration or temperature changes suddenly, and the difference between the virtual line and measured values settles within a predetermined allowable range, and therefore if the measured values of the light emission quantity of ATP light emission reagent of the above item (2) are out of a range of values of adding or subtracting the product of multiplying the value of the light emission quantity by the variation coefficient of measured values of the light emission quantity of ATP light emission reagent of the above item (1), to or from the value of the light emission quantity indicated by the virtual line, by judging that the measured values are not reliable, it is possible to check and remedy in an earlier stage in the event of deterioration or trouble of photomultiplier tube, clogging of nozzles in the dispensing and dividing systems, trouble of the device for adjusting the discharging speed, and other troubles, and thereby the reliability of the inspection results obtained by the inspection method may be maintained.

Generally, a measuring apparatus has its own measuring range, and the dynamic range of a photomultiplier tube used as light emission quantity measuring means is 100 dB or more. If a measured value of light emission quantity of standard ATP sample or a measured value of light emission quantity of captured ATP sample is out of a range capable of maintaining the linearity of the proportional relation, the measured value is obtained if the number of bacterial cells contained in the captured ATP sample is sufficient, but since it exceeds the guaranteed range of relative precision of measured values, if the first peak value of measured values of the light emission quantity of ATP light emission reagent of the above item (4) is more than 10 times of the limit for maintaining the linearity of light emission reaction of the first peak value of measured values of the light emission quantity of ATP light emission reagent of the above item (2), by judging that the inspection is disabled or that the measured values are not reliable, it is possible to confirm that ATP is contained more than the allowable values in the captured ATP sample, and it is possible to check and remedy in an earlier stage in the event of deterioration of performance of ATP eliminating agent, or deterioration or trouble of photomultiplier tube, and thereby the reliability of the inspection results obtained by the inspection method may be maintained.

The average value of light emission quantities of ATP light emission reagent in a predetermined period before injection of standard ATP sample, and the average value of light emission quantities of ATP light emission reagent in a predetermined period after lapse of a specified time by injecting the standard ATP sample are both measurements of background noise, and a large variation thereof suggests abnormality in the hardware such as leak of external disturbance light, and by judging that the measured values are not reliable if the variation coefficient of measured values of the light emission quantity of ATP light emission reagent of the above item (3) is out of a range of 0.5 times to 1.5 times of the variation coefficient of measured values of the light emission reagent of the above item (1), it is possible to check the reagent in an earlier stage, repair the device and improve, and thereby the reliability of the inspection results obtained by the inspection method may be maintained.

If there is no significant difference between the first peak value of measured values of the light emission quantity of captured ATP sample, and the value of background noise as the average value of light emission quantities of the ATP light emission reagent in a predetermined period after termination of light emission reaction of ATP of the standard ATP sample after lapse of a specified time after injection of standard ATP sample, the measurement is under the measurement resolution capacity, and it is reasonable to judge that the measurement is disabled including the measured results in aseptic state, and hence if the first peak value of measured values of the light emission quantity of ATP light emission reagent of the above item (4) is smaller than the sum of adding the standard deviation of measured values of the light emission quantity of ATP light emission reagent of the above item (3) to the average value of measured values of the light emission quantity of ATP light emission reagent of the above item (3), it may be judged that the inspection is disabled or that the measured values are not reliable, and therefore in the event of deterioration of performance of the ATP light emission reagent, the reagent may be checked and improved in an earlier stage, and the reliability of inspection results obtained by the inspection method may be maintained. In addition, by confirming that the captured ATP sample is in aseptic state (it can be confirmed by cultivating with agar culture medium), and it is assured that the capturing position is an aseptic space.

According to the inspection apparatus of airborne floating bacteria of the second aspect of the invention, by measuring the light emission quantity of the ATP light emission reagent about the above-mentioned items (1) to (4) about the ATP light emission reagent by the light emission quantity measuring means of the ATP light emission reagent, and by comparing a measured value of light emission quantity of the ATP light emission reagent and a predetermined theoretical value corresponding to the measured value, the apparatus includes reliability evaluating means for evaluating the reliability of the ATP light emission reagent to be used and the inspection results, and the inspection apparatus of airborne floating bacteria can inform the inspection operator easily of abnormality if there is any abnormality in of the ATP light emission reagent to be used and the inspection results.

**Brief Description of the Drawings**

Fig. 1 shows a schematic configuration diagram of airborne floating bacteria capturing means of an inspection apparatus of airborne floating bacteria of the invention, in which:

(a) is a sectional view of a capturing device, and (b) is an outline view of capturing apparatus.

Fig. 2 is a schematic configuration diagram of the inspection apparatus of airborne floating bacteria of the invention.
Fig. 3 is an outline flowchart of an inspection method of airborne floating bacteria of the invention.
Fig. 4 is a graph showing a light emission quantity of an ATP light emission reagent to be measured in an ATP measuring process of the inspection method.
Fig. 5 shows an allowable range of attenuation rate defined by Rule 3, in which (a) is an explanatory diagram, and (b) is a comparative diagram with actual measured values.
Fig. 6 is a flowchart for analyzing a cause of abnormality by Rule 1.
Fig. 7 is a flowchart for analyzing a cause of abnormality by Rules 2 to 4.
Fig. 8 is a flowchart for analyzing a cause of abnormality by Rules 5 to 8.
Fig. 9 is a flowchart for evaluation of reliability.
Fig. 10 shows an allowable range defined by Rule 4, in which (a) shows an allowable range when calculated by the attenuation rate alone, and (b) shows an allowable range in a corrected case.

**Best Mode for Carrying Out the Invention**

Preferred embodiments of the inspection method of airborne floating bacteria and its method of the invention are described below while referring to the accompanying drawings.

**Embodiment 1**

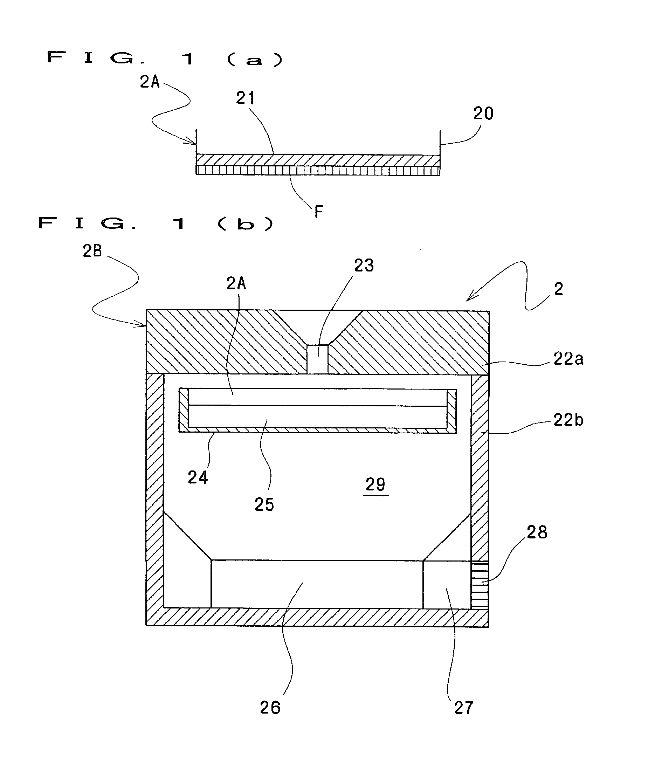

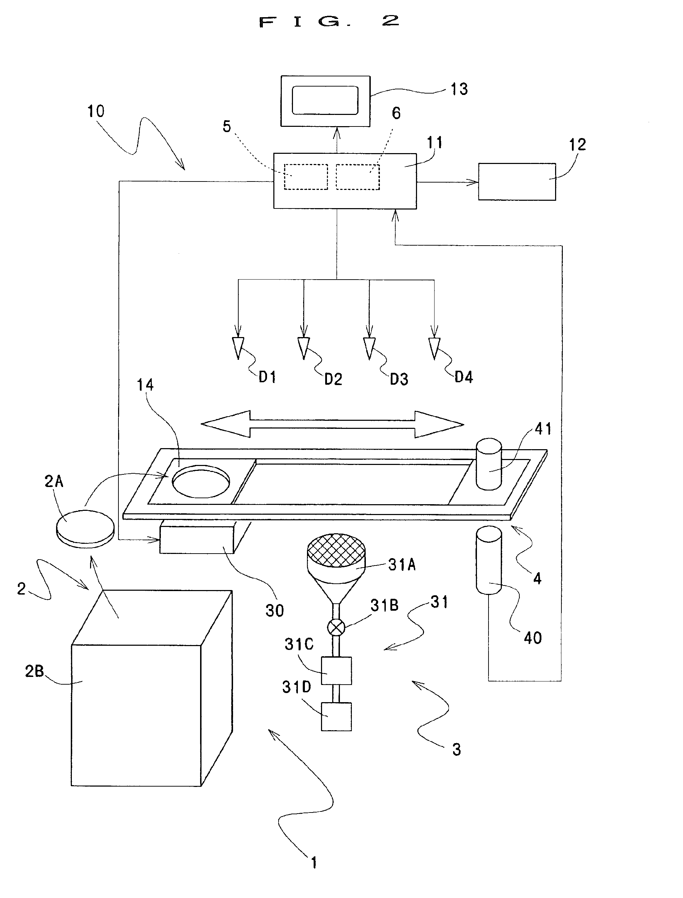

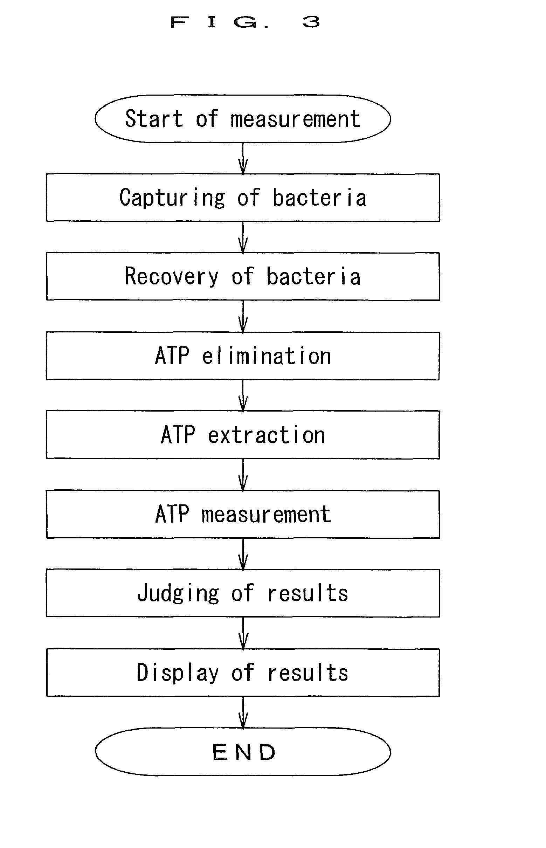

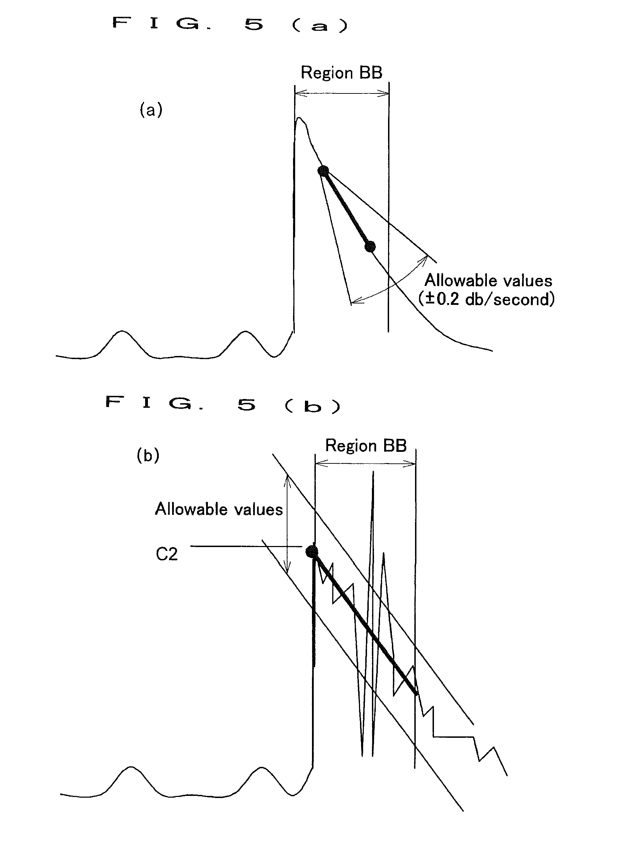

A preferred embodiment of the inspection method of airborne floating bacteria and its apparatus of the invention is shown in Fig. 1 to Fig. 9.
This inspection method of airborne floating bacteria is characterized by performing a biological light emission reaction by using an ATP light emission reagent on the basis of the ATP derived from viable cells in airborne floating bacteria captured in the air contained in an inspection sample, measuring the light emission quantity due to the biological light emission reaction, determining the ATP quantity contained in the inspection sample, and counting the number of cells in the airborne floating bacteria, in which the ATP light emission reagent is evaluated by measuring the light emission quantity of the ATP light emission reagent about the following items (1) to (4), and measured values of light emission quantity of the ATP light emission reagent and predetermined theoretical values corresponding to the measures values are compared, and the reliability of the ATP light emission reagent to be used and the inspection results is evaluated at the same time.

(1) Light emission quantity of ATP light emission reagent in a predetermined period before injection of ATP.

(2) Light emission quantity of ATP light emission reagent in a predetermined period right after injection of specified amount of ATP.

(3) Light emission quantity of ATP light emission reagent in a predetermined period at the termination point of ATP light emission reaction by injecting a specified amount of ATP, and consuming the ATP by ATP light emission reaction after lapse of a predetermined time.

(4) Light emission quantity of ATP light emission reagent in a predetermined period right after injection of ATP derived from viable cells in airborne floating bacteria captured in the air.

An inspection apparatus 1 of airborne floating bacteria to be used in the inspection method of airborne floating bacteria includes airborne floating bacteria capturing means 2 for capturing airborne floating bacteria in the air, inspection sample generating means 3 for generating an inspection sample extracting an ATP derived from viable cells in the airborne floating bacteria captured by the airborne floating bacteria capturing means 2, light emission quantity measuring means 4 of an ATP light emission reagent for performing a biological light emission reaction by using the ATP light emission reagent on the basis of the ATP derived from viable cells in the captured airborne floating bacteria captured in the air contained in the inspection sample, and measuring the light emission quantity due to the biological light emission reaction, and arithmetic means 5 for determining the ATP quantity contained in the inspection sample on the basis of the measured light emission quantity of the ATP light emission reagent, and counting the number of cells in the airborne floating bacteria, further includes reliability evaluating means 6 for evaluating the reliability of the ATP light emission reagent and the inspection results, by comparing between measured values of light emission quantity of the ATP light emission reagent and predetermined theoretical values corresponding to the measures values, by measuring the light emission quantity of the ATP light emission reagent about the above items (1) to (4) about the ATP light emission reagent by the light emission quantity measuring means 4 of the ATP light emission reagent.

The airborne floating bacteria capturing means 2 is composed of a capturing device 2A and a capturing apparatus 2B, and by using the capturing apparatus 2B in which the capturing device 2A is incorporated, airborne floating bacteria can be captured in a capturing carrier 21 (in a gel state in this example) of the capturing device 2A.

The capturing device 2A includes, same as in the prior art, the capturing carrier 21 containing a high polymer for phase transfer between gel and sol at a temperature of 40°C or less, more specifically in a range of 15°C or more to 35°C or less, and a container 20 for accommodating the capturing carrier 21. Besides, by using a filter F in the bottom of the container 20, a series of process after capturing of bacteria can be automated.

The capturing apparatus 2B includes an upper member 22a, a lower member 22b, an intake nozzle 23, a holder 24 for capturing device 2A, a temperature regulating mechanism 25, a suction pump 26, an exhaust duct 27, and an exhaust filter 28, and a gap (space) 29 is formed inside of the apparatus.

In this capturing apparatus 2B, the upper member 22a is detachable from the lower member 22b, and when the upper member 22a is mounted on the lower member 22b to compose the capturing apparatus 2B, the junction of the two becomes airtight, and the communicating part of this capturing apparatus 2B with outside is only the intake nozzle 23 in the upper member 22a and the exhaust filter 28 communicating with the exhaust duct 27 of the suction pump 26 in the lower member 22b.

In the holder 24 for capturing device 2A, and the suction pump 26, the inside of the lower member 22b is disposed, and the gas discharged from a discharge port of the suction pump 26 passes through the exhaust duct 27, filtered by the exhaust filter 28, and is discharged out of the capturing apparatus 2B.

Although not shown in the drawing, the capturing apparatus 2B incorporates a control system and a power supply system for suction pump 26 and temperature regulating mechanism 25, and has operation buttons, handles and others disposed at appropriate positions.

The holder 24 for capturing device 2A is provided with the temperature regulating mechanism 25, and accommodates the capturing device 2A provided detachably, and when the capturing device 2A is accommodated, the upper side of the temperature regulating mechanism 25 contacts with the lower side of the capturing device 2A, and the temperature of the capturing carrier 21 is regulated at 40°C or less, more specifically in a range of 15°C or more to 35°C or less, so that the gel state of the capturing carrier 21 may be maintained.

In the inside of the capturing apparatus 2B, the gap 29 is provided for communicating from the outlet of the intake nozzle 23 to the suction port of the suction pump 26 by way of the capturing device 2A, and it is designed to communicate from the discharge port of the suction pump 26 to the exhaust filter 28 by keeping airtight by means of the exhaust duct 27.

In the capturing apparatus 2B of so-called impactor type as in the embodiment, the flow rate of sample air captured in the air is sufficiently high, and a sample of a specified amount can be sucked in a short time, and airborne floating bacteria can be captured.

In Fig. 1 (b), meanwhile, the intake nozzle 23 has one hole, but the intake nozzle 23 applicable in the invention is not limited to this configuration alone, but various intake nozzles having many small holes may be also employed.

The inspection sample generating means 3 and the light emission quantity measuring means 4 are incorporated in the automatic measuring apparatus 10, and the inspection sample generating means 3 is compose of a mechanism for generating a captured ATP sample containing only ATP derived from viable cells in the airborne floating bacteria, from airborne floating bacteria, debris and others captured in the capturing carrier 21.

More specifically, it is composed of the holder 14 for capturing device 2A mounting the capturing device 2A dismounted from the capturing apparatus 2B, the temperature regulating mechanism 30 for maintaining at a sol phase transfer temperature (for example, 37°C to 40°C), a dispenser D1 for ATP eliminating agent for adding and mixing (ATP eliminating process) an ATP eliminating agent for eliminating the ATP outside of the bacteria or the ATP in the dead bacteria in the sample recovered (bacteria recovery process) from a specified amount of the sample from the sol phase transferred bacterial suspension solution, a filtering mechanism 31 for filtering liquid components containing debris smaller than bacteria, and capturing viable cells in the sample on a filter F, a dispenser D2 for ATP extraction reagent for extracting (ATP extracting process) the ATP contained in the cytoplasm of the filtered viable cells, and a pipetter D3 for ATP recovery for recovering (ATP recovery process) the ATP derived from the viable cells obtained on the filter F. The filtering mechanism 31 is composed by a combination of a suction mechanism composed of a perforated dish holder 31 A, an electromagnetic valve 31 B, a waste liquid trap 31 C, and a suction pump 31D, and the lower side of the filter F.

The light emission quantity measuring means 4 is composed of a dispenser D4 for ATP light emission reagent for injecting an ATP light emission reagent, a photo counter 40 (for example, photomultiplier tube) for measuring the light emission quantity at the time of light emission by the ATP light emission reagent by a biological light emission reaction, and a test tube holder 41 for a test tube (not shown) for injecting and mixing the sample and the reagent.

Herein, the dispensers D1, D2, D3, and D4 are described as individual units, but one dispenser may be used for multiple purposes out of the dispensers D1, D2, D3, and D4, for example, commonly for the dispenser D1 for ATP eliminating agent, the dispenser D2 for ATP extracting reagent, and the dispenser D4 for ATP light emission reagent (the combination of the dispensers is not particularly specified).

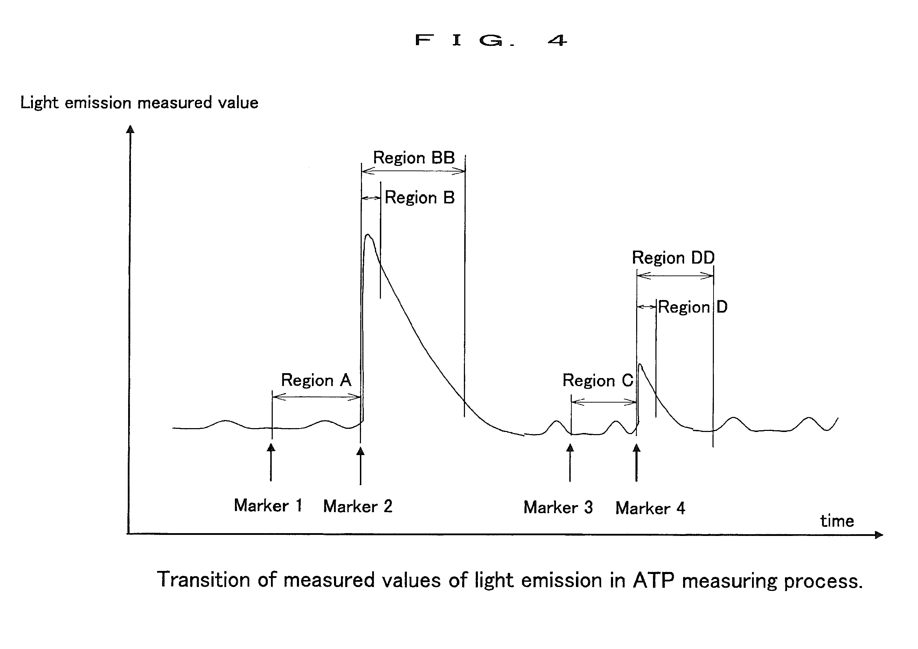

The light emission quantity measuring means 4 measures the light emission quantity of each ATP light emission reagent in the ATP measuring process shown in Fig. 4.

The steps of measurement of light emission quantity in the ATP measuring process are specifically described below.

Marker 1 indicates a measurement starting time of light emission quantity (background noise) of the ATP light emission reagent before injection of a standard ATP sample.

Region A from marker 1 to marker 2 is a region for measuring the background noise immediately before measurement of light emission quantity of the standard ATP sample of a known ATP amount, and the average value of measured values in region A is defined as C1 same as in the prior art, and the standard deviation of measured values in region A is defined as SD1.

At the timing of marker 2, light emission reaction of the standard ATP sample begins.

Region B indicates a period of measurement of light emission quantity of standard ATP sample, and the first peak value (crest value) of region B is supposed to be C2.

Region BB indicates an attenuation monitoring period of light emission quantity in measurement of light emission quantity of the standard ATP sample including region B.

Marker 3 indicates a measurement starting time of light emission quantity (background noise) of the ATP light emission reagent before injection of a standard ATP sample, after measurement of light emission quantity of the standard ATP sample.

Marker 4 indicates a measurement starting time of light emission quantity of captured ATP sample.

Region C from marker 3 to marker 4 indicates a background noise measuring region before start of measurement of light emission quantity of the captured ATP sample, and the average value of measured values in region C is defined as C3, and the standard deviation of measured values in region C is defined as SD3.

Region D indicates a period of measurement of light emission quantity of standard ATP sample, and the first peak value (crest value) of region D is supposed to be C4.

Region DD indicates an attenuation monitoring period of light emission quantity in measurement of light emission quantity of the captured ATP sample including region D.

The period of each region is, for example, 120 seconds for region A, 10 seconds for region B, 120 seconds for region BB, 120 seconds for region C, and 10 seconds for region D.

In the period of region DD, in an ordinary measuring range of measurement of captured ATP sample, if the ATP amount of the captured ATP sample is smaller than the standard ATP sample, the period of region DD is shorter than the period of region BB, being specifically 20 to 30 seconds.

On the other hand, when the ordinary measuring range is specified in the case the ATP amount of the captured ATP sample is nearly same as the standard ATP sample or more than the standard ATP sample, the period of region DD is same as the period of region BB.

Herein, the ordinary measuring range varies with the environment of measurement of airborne floating bacteria, and, for example, in the case of measurement in manufacturing environments of aseptic pharmaceutical preparations, the ATP converted value is 0 to hundreds of amol, and 0 to hundreds of amol becomes an ordinary measuring range. At this time, the standard ATP sample is 500 amol.

When desired to obtain an ATP conversion value of about 1000 to tens of thousand of amol in other than manufacturing environments of aseptic pharmaceutical preparations, for example, in measurement in manufacturing environments of foods and related products, it is intended to inspect by using a standard ATP sample of about 10,000 amol.

The arithmetic means 5 is designed to count the number of cells in the airborne floating bacteria by using formula 1 and formula 2, and in this inspection apparatus 1 of airborne floating bacteria, prior to arithmetic processing by the arithmetic means 5 using formula 1 and formula 2, the reliability of ATP light emission reagent and inspection results is evaluated by using the reliability evaluating means 6, and when the measured values are judged to be reliable, the number of cells in the airborne floating bacteria is counted, and the result is displayed in the display means 13 such as a display screen by means of the control device 11.

The dispenser D1 for ATP eliminating agent, the dispenser D2 for ATP extracting reagent, the pipetter D3 for ATP recovery, and the dispenser D4 for ATP light emission reagent are individually provided with automatic mechanism for dispensing and pipetting the reagents by control signals from the control device 11.

The electromagnetic valve 31 B and the suction pump 31D are controlled by the control device 11, and are designed to manage the filtering process.

The temperature regulating mechanism 30 is controlled by the control device 11, and is designed to control the temperature in various inside parts of the automatic measuring apparatus 10, especially the temperature of the holder 14 for capturing device 2A at 37°C or more to 40°C or less.

The photomultiplier tube 40 measures the light emission quantity (measuring the intensity of light emission as the number of photons) of the ATP light emission reagent generated in a test tube (not shown) installed in a test tube holder 41, and issues the measured value to the control device 11.

Prior to start of use of the automatic measuring apparatus 10, the inside of the automatic measuring apparatus 10 is sterilized by using a sterilizing cleaning mechanism 12, and ozone gas (when ultraviolet ray sterilizing method is employed) and particulates are eliminated.

During use of the automatic measuring apparatus 10, it is also preferred to operate while eliminating ozone gas and particulates by using the sterilizing cleaning mechanism 12.

The reliability evaluating means 6 for evaluating the reliability of the ATP light emission reagent to be used and the inspection results compares the measured values of the light emission quantity of the ATP light emission reagent of the above item (1) to (4) measured about the ATP light emission reagent by the light emission quantity measuring means 4, with predetermined theoretical values corresponding to the measured values in the control device 11 (or arithmetically operated by the arithmetic means 5), and judges that the reliability is absent when the measured values are out of a range of theoretical values, and more specifically the reliability is evaluated according to eight rules as specified below.

<Rule 1>

If the variation coefficient of measured values of light emission quantity of the ATP light emission reagent of the above item (1) is more than 40%, it is judged that the measured values are not reliable.

That is, heat noise of a photomultiplier tube used as the light emission quantity measuring means 4 for measuring the light emission quantity of an ATP light emission reagent is a finite value, and is nearly a constant value in a state free from external disturbance light, and the variation coefficient obtained by dividing the standard deviation of light emission quantity of ATP light emission reagent in a predetermined period before injection of ATP by the average number is a normal value in a range of 30 to 40% in measurement of photon counting method for output of one pulse by incident of one photon, in a general combination of a photomultiplier tube, an amplifier, and a comparator, and hence it is judged that the measured values are not reliable if the variation coefficient (SD1/C1) of the measured values (region A) of the light emission quantity of the ATP light emission reagent of the above item (1 ) is more than 40%.

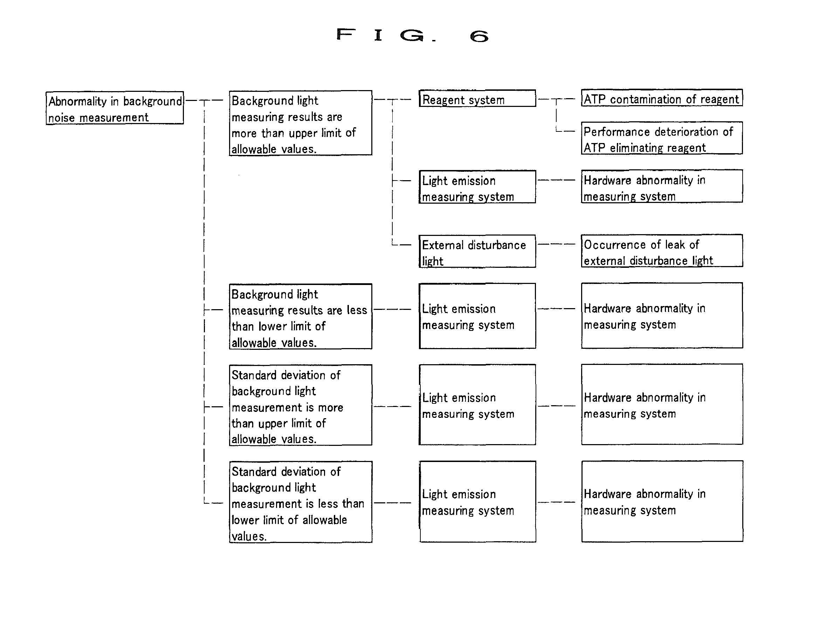

Factors of judging not reliable according to Rule 1 include, as shown in Fig. 6, contamination of the reagent, troubles of the light emission measuring system, and others, and it is possible to maintain the reliability of inspection results obtained by the inspection method by checking, repairing and improving in an earlier stage, in the event of deterioration or trouble of photomultiplier tube, abnormality of hardware of measurement, presence or absence of contamination of ATP light emission reagent by ATP, or deterioration of performance of ATP eliminating reagent.

<Rule 2>

If the balance of subtracting the average value of measured values of the light emission quantity of ATP light emission reagent of the above item (1) from the first peak value of measured values of the light emission quantity of ATP light emission reagent of the above item (2) is out of a range of -30% to +30% of the theoretical value of light emission quantity of a specified amount of ATP, it may be judged that the measured values are not reliable.

That is, the first peak value (C2) of the light emission quantity of the ATP light emission reagent in a predetermined period (region B) right after injection of the standard ATP sample is the measured value of the light emission quantity of the sample adjusted to a known ATP content, and if it is out of a range of - 30% to +30% of the theoretical values, in consideration of an error of 20% of concentration of standard ATP sample, and a variation of about 10% due to aging deterioration or reaction temperature of standard ATP standard, it is judged that the measured values are not reliable.

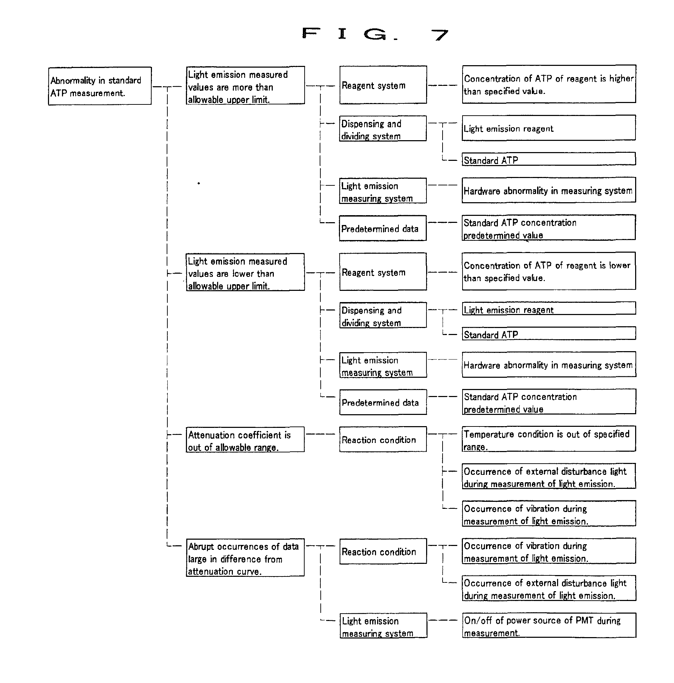

Factors of judging not reliable according to Rule 2 include, as shown in Fig. 7, the concentration of the standard ATP sample being out of the specified value, or troubles of the light emission measuring system, and others, and it is possible to maintain the reliability of inspection results obtained by the inspection method by checking, repairing and improving in an earlier stage, in the event of deterioration or trouble of photomultiplier tube, abnormality of hardware of measurement, or concentration checking of ATP light emission reagent.

<Rule 3>

If the attenuation rate calculated by dividing the value of 10 times of common logarithm of the ratio of measured values at two points arbitrarily selected from measured values of the light emission quantity of ATP light emission reagent of the above item (2) by the time difference of the two points is out of a range of -0.2 dB/second to +0.2 dB/second of the theoretical value of attenuation rate of light emission quantity of a specified amount of ATP, it may be judged that the measured values are not reliable.

That is, the light emission quantity of the ATP light emission reagent in a predetermined period right after injection of standard ATP sample begins to attenuate gradually, and its attenuation rate is an intrinsic value (theoretical value) when the concentration and amount of the standard ATP sample are constant, and if the attenuation rate calculated by dividing the logarithmic difference of measured values at arbitrarily selected two points (for example, start and end of a predetermined period) by the time difference of the two points is out of a range of -0.2 dB/second to +0.2 dB/second of the theoretical value of attenuation rate of light emission quantity of a specified amount of ATP, it is designed to be judged that the measured values are not reliable.

Factors of judging not reliable according to Rule 3 include, as shown in Fig. 7, the reaction condition such as temperature condition out of the specified range, occurrence of external disturbance light during measurement of light emission quantity, or occurrence of vibration during measurement of light emission quantity, and others, and it is possible to maintain the reliability of inspection results obtained by the inspection method by checking, repairing and improving in an earlier stage, in the event of abnormality of reaction conditions, such as the temperature condition out of the specified range, occurrence of external disturbance light during measurement of light emission quantity, occurrence of vibration during measurement of light emission quantity, and others.

<Rule 4>

If the measured values of the light emission quantity of ATP light emission reagent of the above item (2) are out of a range of values of adding or subtracting the product of multiplying the value of the light emission quantity by the variation coefficient of measured values of the light emission quantity of ATP light emission reagent of the above item (1 ), to or from the value of the light emission quantity indicated by a virtual line having an inclination of the attenuation rate calculated by separating the measured values (region BB) of the light emission quantity of ATP light emission reagent of the above item (2) into two regions, a first half and a second half, and dividing the average of two values of the average of the logarithm of each measured value of the first half and the average of the logarithm of each value of the second half, by the time difference of 1/2 of the time of region BB, that is, between the first-half period and the second half period, it may be judged that the measured values are not reliable. The virtual line is calculated on the basis of attenuation rate K (dB/s) expressed in formula 3, and estimated value Cn (virtual line estimating the attenuation from peak of C2 after lapse of n seconds) expressed in formula 4.

$$K = 10 \times (\log(C_{2R}/C_{2F})) / Ts \qquad \text{(formula 3)}$$

K : attenuation rate (dB/s)
$C_{2F}$ : average value of first-half data
$C_{2R}$: average value of second-half data
Ts: time difference of first-half data and second-half data (1/2 of period of period BB)
log: common logarithm

$$Cn = C2 \times 10^{(Kn/10)} \qquad \text{(formula 4)}$$

That is, the light emission quantity of the ATP light emission reagent in a predetermined period right after injection of standard ATP begins to deteriorate gradually and stably unless ATP is newly supplied after start of reaction, or the vibration or temperature changes suddenly, and the difference between the virtual line and measured values settles within a predetermined allowable range, and therefore if the measured values (region BB) of the light emission quantity of ATP light emission reagent of the above item (2) are out of a range of values of adding or subtracting the product of multiplying the value of the light emission quantity by the variation coefficient (SD1/C1) of measured values of the light emission quantity of ATP light emission reagent of the above item (1), to or from the value of the light emission quantity indicated by the virtual line, it is judged that the measured values are not reliable.

Factors of judging not reliable according to Rule 4 include, as shown in Fig. 7, occurrence of external disturbance light as reaction condition during measurement of light emission quantity, or occurrence of vibration during measurement of light emission quantity, and others, and may also include on/off trouble during measurement of power source of the photomultiplier tube as light emission quantity measuring means 4, and it is possible to maintain the reliability of inspection results obtained by the inspection method by checking, repairing and improving in an earlier stage, in the event of abnormality of reaction conditions, such as deterioration or trouble of the photomultiplier tube.

<Rule 5>

If the measured values of the light emission quantity of ATP light emission reagent of the above item (4) are out of a range of values of adding or subtracting the product of multiplying the value of the light emission quantity by the variation coefficient of measured values of the light emission quantity by ATP light emission reagent of the above item (1 ), to or from the value of the light emission quantity indicated by a virtual line having an inclination obtained by multiplying the attenuation rate calculated by dividing the logarithmic difference of measured values at two points arbitrarily selected from measured values of the light emission quantity of ATP light emission reagent of the above item (2), by the difference between the first peak value and the average of the light emission quantitaty of the ATP light amission reagent in a subsequent predetermined period, by passing through a point of a first peak value of measured values of the light amission quantitaty of ATP light emission reagent of the above item (4) by the time difference of the two points, it may be judged that the measured values are not reliable.

That is, the light emission quantity of the light emission reagent of the standard ATP is judged by Rule 4 as mentioned above, but if Rule 4 is applied to the ATP light emission of the captured sample, it is necessary to permit a wider judging allowable range. In the standard ATP, the intensity of the light emission is about 10,000 to 20,000 cps, whereas the noise component of the background light is about 100 to 200 cps, and in the calculation of the attenuation rate, presence or absence of correction of the noise component of the background light is not a big error in the calculation of the attenuation rate. If the attenuation rate involves an error of about 1%, if the allowable range is in the setting of 20%, a calculation error of 1% may be allowed. However, in the captured ATP sample, a rule is demanded to be applicable in a range from the limiting light emission having a significant difference from the noise component of the background light to the to an intense light emission of the light emission intensity of the standard ATP light emission.
Accordingly, in the measured value (region DD) of the ATP light emission reagent, the following rule is applied.
In the calculation (formula 2) of the ATP conversion amount, the difference between the first peak value C4 of region DD including region D and the average value C3 of region C is calculated as the ATP derived from the viable cells of the captured sample.
Similarly, the differential portion of the first peak value C4 and the average value C3 of region C is obtained as an effective light emission intensity, and the product obtained by multiplying the attenuation rate determined in Rule 4 by (C4-C3) is determined as an inclination, the value by adding C3 as the noise component is obtained as a virtual line (a virtual line assuming attenuation after n seconds from the peak of C4), and formula 5 is calculated.

$$Cn = (C4-C3) \times 10^{(Kn/10)} + C3 \qquad \text{(formula 5)}$$

If out of a range of values adding or subtracting the product of multiplying by the variation coefficient (SD1/C1) of measured values of the light emission quantity of ATP light emission reagent of the above item (1) to this estimated value of the virtual line, it is judged that the measured values are not reliable.

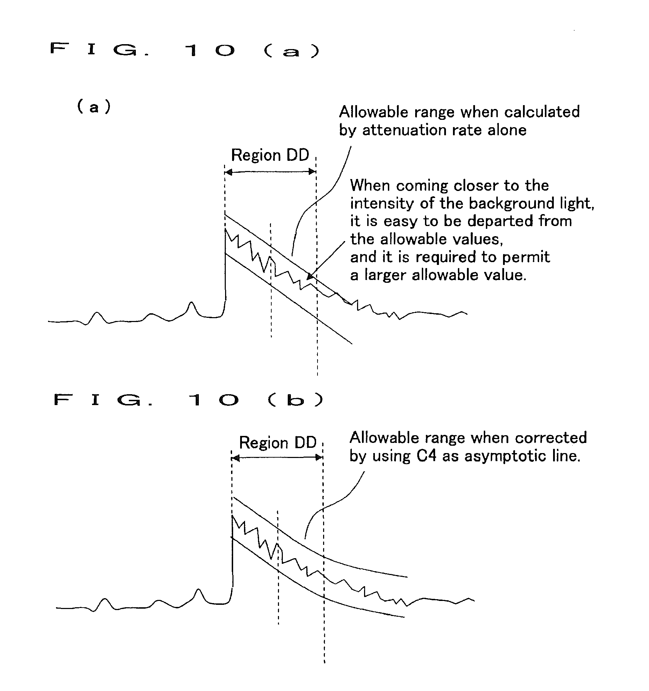

Fig. 10 (a) shows an allowable range calculated by the attenuation rate alone, and Fig. 10 (b) shows an allowable range when corrected by using C4 as an asymptotic line.

The value estimated by the attenuation coefficient alone is a straight line with a specific inclination, and when the light emission quantity is small, as the time elapses, the value converges on C3 which is the noise component. Accordingly, by correcting the straight line of a specific inclination so as to converge on the asymptotic line, the estimation error is decreased in a region lowered in the intensity of light emission, and it is possible to judge with a small allowable value.

Factors of judging not reliable according to Rule 5 include, as shown in Fig. 7, occurrence of external disturbance light as reaction condition during measurement of light emission quantity, or occurrence of vibration during measurement of light emission quantity, and others, and may also include occurrence of abnormality in the discharge speed of dispensing nozzle in the dispensing and dividing system, and it is possible to maintain the reliability of inspection results obtained by the inspection method by checking, repairing and improving in an earlier stage, in the event of deterioration or trouble of the photomultiplier tube, clogging of the nozzles in the dispensing and dividing system, or trouble in the device for adjusting the discharge speed.

In judgment according to Rule 5, in consideration of ordinary measuring range, the variation coefficient to be multiplied to the value of the light emission quantity can be changed.

If the ordinary measuring range is large than in the standard ATP sample (low sensitivity measurement), the variation coefficient for determining the allowable value of region DD is nearly same as in region BB (SD1/C1) same as mentioned above.

On the other hand, if the ordinary range is in a smaller region than in the standard ATP sample (high sensitivity measurement), the variation coefficient for determining the allowable value of region DD is preferred to be selected so as to be sufficiently larger than the standard deviation SD3 of the background noise in region C.

This is because, in the period of region DD, the attenuation of light emission quantity converges on an extended line from C4 to C3, along an asymptotic line of an extended line from C3, but in ATP measurement at high sensitivity, the ATP is consumed in a short time, and the light emission is not continued in the period of region DD, and the difference from the background noise tends to be a small measured value, and hence in measurement at high sensitivity, by introducing a correction calculation by taking the attenuation in region BB and region C as an asymptotic line, it is possible to judge without widening the allowable values of region DD, so that it is applicable to both high-sensitivity measurement low-sensitivity measurement.

<Rule 6>

If the first peak value of measured values of the light emission quantity of ATP light emission reagent of the above item (4) is more than 10 times of the limit for maintaining the linearity of light emission reaction of the first peak value of measured values of the light emission quantity of ATP light emission reagent of the above item (2), it may be judged that the inspection is disabled or that the measured values are not reliable.

That is, generally, there is a measuring range in a measuring instrument, and the dynamic range of the photomultiplier tube as the light emission quantity measuring means 4 is 100 dB or more, and if the measured value of light emission quantity of the standard ATP sample and the measured value of light emission quantity of the captured ATP sample surpass a range capable of maintaining the linearity of the proportional relationship, there is no abnormality in the measured values as far as the number of bacterial cells contained in the captured ATP sample is sufficient, but since it exceeds the guarantee range of precision of measured results, if the first peak value (C4) of measured values of the light emission quantity of ATP light emission reagent of the above item (4) is more than 10 times of the limit for maintaining the linearity of light emission reaction of the first peak value (C2) of measured values of the light emission quantity of ATP light emission reagent of the above item (2), it may be judged that the inspection is disabled or that the measured values are not reliable.

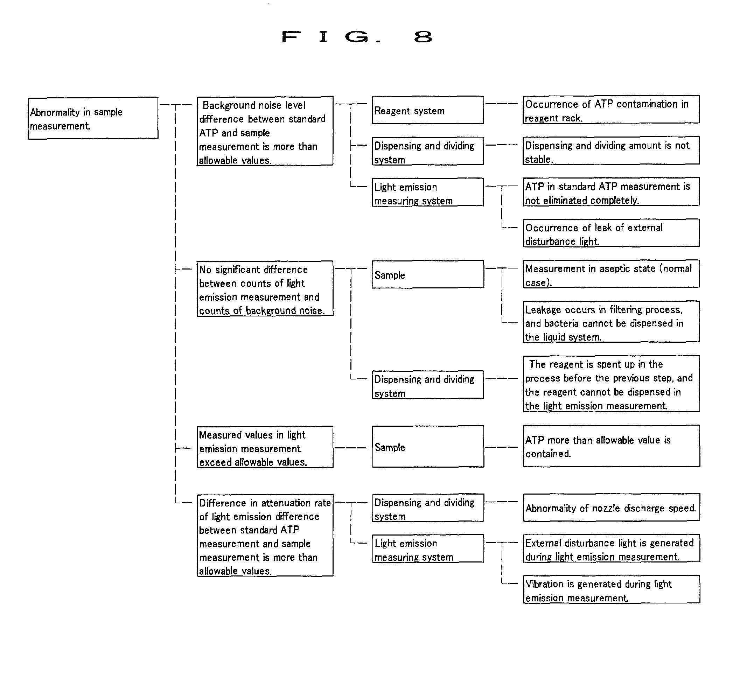

Factors of judging not reliable according to Rule 6 are shown in Fig. 8, and main factors are a high content of ATP more than an allowable value in the captured ATP sample, abnormality in the measuring system, and abnormality in the captured ATP sample (deterioration of performance of ATP eliminating reagent), and it is possible to maintain the reliability of inspection results obtained by the inspection method by checking, repairing and improving in an earlier stage, in the event of deterioration or trouble of the photomultiplier tube, etc.

<Rule 7>

If the variation coefficient of measured values of the light emission quantity of ATP light emission reagent of the above item (3) is out of a range of 0.5 times to 1.5 times of the variation coefficient of measured values of the light emission reagent of the above item (1), it may be judged that the measured values are not reliable.

That is, the average value of light emission quantities of the ATP light emission reagent in a predetermined period before injection of standard ATP sample, and the average value of light emission quantities of the ATP light emission reagent in a predetermined period after lapse of a predetermined time following the injection of standard ATP sample are both measurement of background noise, and when varying significantly, it is assumed that there is some abnormality, such as leak of external disturbance light or trouble of hardware, and therefore if the variation coefficient (SD3/C3) of measured values of the light emission quantity of ATP light emission reagent of the above item (3) is out of a range of 0.5 times to 1.5 times of the variation coefficient (SD1/C1) of measured values of the light emission reagent of the above item (1), it may be judged that the measured values are not reliable.

Factors of judging not reliable according to Rule 7 include, as shown in Fig. 8, contamination of ATP in the reagent rack, instability of dispensing and dividing amounts, abnormality in the light emission measuring system, and others, and it is possible to maintain the reliability of inspection results obtained by the inspection method by checking the reagents, and repairing and improving the devices in an earlier stage.

<Rule 8>

If the first peak value of measured values of the light emission quantity of ATP light emission reagent of the above item (4) is smaller than the sum of adding the standard deviation of measured values of the light emission quantity of ATP light emission reagent of the above item (3) to the average value of measured values of the light emission quantity of ATP light emission reagent of the above item (3), it may be judged that the inspection is disabled or that the measured values are not reliable.

That is, it is a measurement under the measurement resolution if there is no significant difference between the first peak value of measured values of the light emission quantity of the captured ATP sample, and the background noise, that is, the average value of light emission quantities of the ATP light emission reagent in a predetermined period after lapse of a preset time following the injection of standard ATP sample, and it is reasonable to judge that the measurement is disabled, including the measurement results in an aseptic state, and therefore if the first peak value (C4) of measured values of the light emission quantity of ATP light emission reagent of the above item (4) is smaller than the sum of adding the standard deviation (SD3) of measured values of the light emission quantity of ATP light emission reagent of the above item (3) to the average value (C3) of measured values of the light emission quantity of ATP light emission reagent of the above item (3), it may be judged that the inspection is disabled or that the measured values are not reliable.

Factors of judging not reliable according to Rule 8 include, as shown in Fig. 8, measurement in an aseptic state, occurrence of leak in the filtering process, failure in dispensing of bacteria in the liquid system, and others, and therefore by inspecting for presence or absence of leak in the filtering process, if a leak is found, it is judged that the filtering process is abnormal, and if no leak is found, it is judged that the state is aseptic below the measurement limits. Besides, other possible factors include deterioration of the ATP light emission reagent, and abnormality of the light emission measuring system, and it is possible to maintain the reliability of inspection results obtained by the inspection method by checking the reagents, and repairing and improving the devices in an earlier stage.

In addition to the eight rules explained above, it is also preferred to inspect for leak in the filtering process. That is, as explained in relation to Rule 8, if the captured airborne floating bacteria should leak in the filtering process, in judgment by Rule 8, same as in measurement in aseptic state, there is no significant difference between the first peak value of measured values of the light emission quantity of the captured ATP sample, and the background noise, that is, the average value of light emission quantities of the ATP light emission reagent in a predetermined period after lapse of a preset time following the injection of standard ATP sample, and moreover if part of bacteria leaks due to leakage in the filtering process, the number of bacterial cells of the inspection results becomes smaller than the actual number of bacterial cells, and the reliability of the inspection is substantially spoiled.

The leakage inspection in the filtering process includes a bacteria recovery process of obtaining a bacterial suspension solution (sample) by phase transfer of the capturing carrier 21 from gel to sol, an ATP eliminating process of eliminating extracellular ATP in the sample or ATP in the dead bacterial cells, and a filtering process of filtering the components in the sample, for sorting out debris smaller than bacteria, from a mixed state of viable cells in the airborne floating bacteria and debris, and it is intended to prevent the bacteria from being discharged together with the debris, and this inspection is carried out simultaneously with the periodic checking of the inspection apparatus 1.

The leakage inspection in the filtering process is not particularly specified as far as it is possible to inspect whether the viable cells in the sample can be captured on the filter, and in the embodiment, the filtering process is executed by using a filter loaded with bacteria such as Bacillus subtilis, and if the loaded Bacillus subtilis and other ATP are not detected, it is judged that they are filtered in the filtering process.

When it is judged that the bacteria captured in the filtering process are leaking, the members of the filtering mechanism 31 used in the filtering process are checked, and especially the mounting precision of the perforated dish holder 31A, the perforated dish (not show), and the filer F is checked, the suction force of the suction pump 31D is adjusted, and leak countermeasures in the filtering process are executed, and it is possible to maintain the reliability of inspection results obtained in this inspection method.

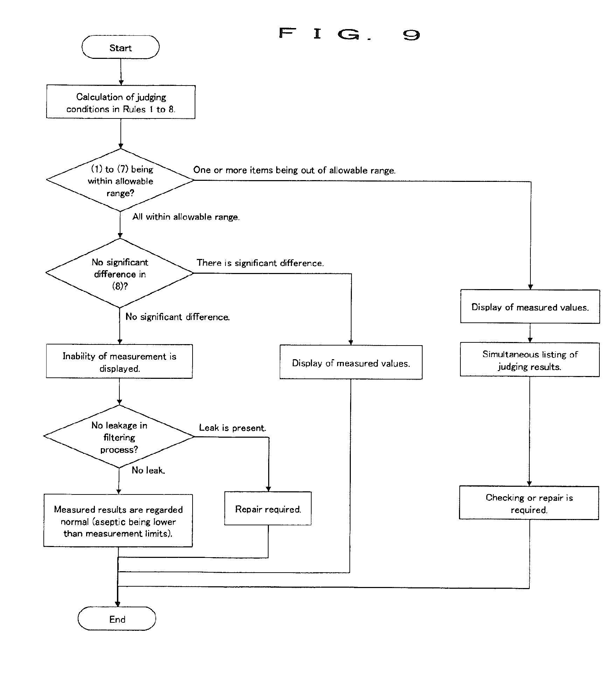

The judging rules and the leakage inspection in the filtering process described so far are explained according to the flowchart in Fig. 9, in which by judging with reliability evaluating means 6, the reliability of ATP light emission reagent to be used and inspection results is evaluated from plural factors including the trouble of the measuring instrument and abnormality of the measured values, and it is possible to maintain the reliability of inspection results obtained by the inspection method by checking the reagents, and repairing and improving the devices in an earlier stage.

So far, the inspection method of airborne floating bacteria and its apparatus of the invention are described on the basis of the preferred embodiment thereof, but the invention is not limited to the illustrated configuration of the embodiment, and may be changed and modified in various forms within a scope not departing from the true spirit thereof. The above embodiments of the invention as well as the appended claims and figures show multiple characterizing features of the invention in specific combinations. The skilled person will easily be able to consider further combinations or subcombinations of these features in order to adapt the invention as defined in the in the claims to his specific needs.

**Industrial Applicability**

The inspection method of airborne floating bacteria and its apparatus of the invention are intended to measure the light emission quantity by a biological light emission reaction, determine the ATP quantity contained in the inspection sample, and count the number of cells in the airborne floating bacteria, and are hence characterized by evaluating the reliability of the ATP light emission reagent to be used and the inspection results, and are therefore applied widely in the inspection method of airborne floating bacteria and its apparatus.

**Description of the Reference Numerals**

1    Inspection apparatus of airborne floating bacteria

2    Airborne floating bacteria capturing means

3    Inspection sample generating means

4    Light emission quantity measuring means

5    Arithmetic means

6    Reliability evaluating means

**Claims**

1.    An inspection method of airborne floating bacteria comprising the steps of:

- performing a biological light emission reaction by using an ATP light emission reagent on the basis of the ATP derived from viable cells in captured airborne floating bacteria,
- measuring the light emission quantity due to the biological light emission reaction,
- determining the ATP quantity contained in the inspection sample, and counting the number of cells in the airborne floating bacteria,

wherein the ATP light emission reagent is evaluated by measuring the light emission quantity of the ATP light emission reagent about the following items (1 ) to (4), and measured values of light emission quantity of the ATP light emission reagent and predetermined theoretical values corresponding to the measured values are compared, and the reliability of the ATP light emission reagent to be used and the inspection results is evaluated at the same time, wherein said items (1) to (4) are:

(1) Light emission quantity of ATP light emission reagent in a predetermined period before injection of ATP;
(2) Light emission quantity of ATP light emission reagent in a predetermined period right after injection of specified amount of ATP;
(3) Light emission quantity of ATP light emission reagent in a predetermined period at the termination point of ATP light emission reaction by injecting a specified amount of ATP, and consuming the ATP by ATP light emission reaction after lapse of a predetermined tim; and

(4) Light emission quantity of ATP light emission reagent in a predetermined period right after injection of ATP derived from viable cells in airborne floating bacteria captured in the air.

2. The inspection method of airborne floating bacteria according to claim 1, wherein if the variation coefficient of measured values of the light emission quantity of ATP light emission reagent of the above item (1) is more than 40%, it is judged that the measured values are not reliable.

3. The inspection method of airborne floating bacteria according to claim 1 or 2, wherein if the balance of subtracting the average value of measured values of the light emission quantity of ATP light emission reagent of the above item (1) from the first peak value of measured values of the light emission quantity of ATP light emission reagent of the above item (2) is out of a range of -30% to +30% of the theoretical value of light emission quantity of a specified amount of ATP, it is judged that the measured values are not reliable.

4. The inspection method of airborne floating bacteria according to one of claims 1 - 3, wherein if the attenuation rate calculated by dividing the logarithmic difference of measured values at two points arbitrarily selected from measured values of the light emission quantity of ATP light emission reagent of the above item (2) by the time difference of the two points is out of a range of - 0.2 dB/second to +0.2 dB/second of the theoretical value of attenuation rate of light emission quantity of a specified amount of ATP, it is judged that the measured values are not reliable.

5. The inspection method of airborne floating bacteria according to one of claims 1 - 4, wherein if the measured values of the light emission quantity of ATP light emission reagent of the above item (2) are out of a range of values of adding or subtracting the product of multiplying the value of the light emission quantity by the variation coefficient of measured values of the light emission quantity of ATP light emission reagent of the above item (1), to or from the value of the light emission quantity indicated by a virtual line having an inclination of the attenuation rate calculated by separating measured values of the light emission quantity of ATP light emission reagent of the above item (2) into two regions, a first half and a latter half, and dividing the average value of the two values of the average value of logarithm of each measured value of the first half and the average of logarithm of each measured value of the second half, by the time difference of the first half region and the second half region, it may be judged that the measured values are not reliable.

6. The inspection method of airborne floating bacteria according to one of claims 1 - 5, wherein if the measured values of the light emission quantity of ATP light emission reagent of the above item (4) are out of a range of values of adding or subtracting the product of multiplying the value of the light emission quantity by the variation coefficient of measured values of the light emission quantity of ATP light emission reagent of the above item (1), to or from the value of the light emission quantity indicated by a virtual line having an inclination obtained as an inclination by multiplying the attenuation rate calculated by separating measured values of the light emission quantity of ATP light emission reagent of the above item (2) into two regions, a first half and a latter half, and dividing the average value of the two values of the average value of logarithm of each measured value of the first half and the average of logarithm of each measured value of the second half, by the time difference of the first half region and the second half region, and by adding the average of the light

7. The inspection method of airborne floating bacteria according to one of the preceding claims, wherein if the first peak value of measured values of the light emission quantity of ATP light emission reagent of the above (4) is more than 10 times of the limit for maintaining the linearity of light emission reaction of the first peak value of measured values of the light emission quantity of ATP light emission reagent of the above (2), it is judged that the inspection is disabled or that the measured values are not reliable.

8. The inspection method of airborne floating bacteria according to one of the preceding claims, wherein if the variation coefficient of measured values of the light emission quantity of ATP light emission reagent of the above (3) is out of a range of 0.5 times to 1.5 times of the variation coefficient of measured values of the light emission reagent of the above (1), it is judged that the measured values are not reliable.

9. The inspection method of airborne floating bacteria according to to one of the preceding claims, wherein if the first peak value of measured values of the light emission quantity of ATP light emission reagent of the above (4) is smaller than the sum of adding the standard deviation of measured values of the light emission quantity of ATP light emission reagent of the above (3) to the average value of measured values of the light emission quantity of ATP light emission reagent of the above (3), it is judged that the inspection is disabled or that the measured values are not reliable.

**10.** An inspection apparatus of airborne floating bacteria comprising airborne floating bacteria capturing means for capturing airborne floating bacteria in the air, inspection sample generating means for generating an inspection sample extracting an ATP derived from viable cells in the airborne floating bacteria captured by the airborne floating bacteria capturing means, light emission quantity measuring means of an ATP light emission reagent for performing a biological light emission reaction by using the ATP light emission reagent on the basis of the ATP derived from viable cells in the captured airborne floating bacteria captured in the air contained in the inspection sample, and measuring the light emission quantity due to the biological light emission reaction, and arithmetic means for determining the ATP quantity contained in the inspection sample on the basis of the measured light emission quantity of the ATP light emission reagent, and counting the number of cells in the airborne floating bacteria, further comprising reliability evaluating means for evaluating the reliability of the ATP light emission reagent and the inspection results, by comparing between measured values of light emission quantity of the ATP light emission reagent and predetermined theoretical values corresponding to the measures values, by measuring the light emission quantity of the ATP light emission reagent about the following items (1 ) to (4) about the ATP light emission reagent by the light emission quantity measuring means of the ATP light emission reagent, wherein said items (1) to (4) are:

(1) Light emission quantity of ATP light emission reagent in a predetermined period before injection of ATP;
(2) Light emission quantity of ATP light emission reagent in a predetermined period right after injection of specified amount of ATP;
(3) Light emission quantity of ATP light emission reagent in a predetermined period at the termination point of ATP light emission reaction by injecting a specified amount of ATP, and consuming the ATP by ATP light emission reaction after lapse of a predetermined time; and
(4) Light emission quantity of ATP light emission reagent in a predetermined period right after injection of ATP derived from viable cells in airborne floating bacteria captured in the air.

F I G. 1 (a)

F I G. 1 (b)

F I G . 2

F I G. 3

```
        ┌──────────────────────────────┐
        │     Start of measurement     │
        └──────────────┬───────────────┘
                       ↓
        ┌──────────────────────────────┐
        │     Capturing of bacteria    │
        └──────────────┬───────────────┘
                       ↓
        ┌──────────────────────────────┐
        │     Recovery of bacteria     │
        └──────────────┬───────────────┘
                       ↓
        ┌──────────────────────────────┐
        │       ATP elimination        │
        └──────────────┬───────────────┘
                       ↓
        ┌──────────────────────────────┐
        │        ATP extraction        │
        └──────────────┬───────────────┘
                       ↓
        ┌──────────────────────────────┐
        │       ATP measurement        │
        └──────────────┬───────────────┘
                       ↓
        ┌──────────────────────────────┐
        │      Judging of results      │
        └──────────────┬───────────────┘
                       ↓
        ┌──────────────────────────────┐
        │      Display of results      │
        └──────────────┬───────────────┘
                       ↓
        ┌──────────────────────────────┐
        │             END              │
        └──────────────────────────────┘
```

Light emission measured value

Transition of measured values of light emission in ATP measuring process.

EP 2 343 381 A1

EP 2 343 381 A1

F I G. 5 (a)

(a)

Region BB

Allowable values
(±0.2 db/second)

F I G. 5 (b)

(b)

Region BB

Allowable values

C2

22

# F I G. 6

```
┌─────────────────────┐   ┌─────────────────────┐   ┌─────────────────┐   ┌──────────────────────────┐
│Abnormality in       │───┬──│Background light     │───┬──│Reagent system   │───┬──│ATP contamination of reagent│
│background           │   │  │measuring results are│   │  └─────────────────┘   └──────────────────────────┘
│noise measurement    │   │  │more than upper      │   │                        ┌──────────────────────────┐
└─────────────────────┘   │  │limit of allowable   │   │                      └──│Performance deterioration │
                          │  │values.              │   │                         │of ATP eliminating reagent│
                          │  └─────────────────────┘   │                         └──────────────────────────┘
                          │                            │  ┌─────────────────┐   ┌──────────────────────────┐
                          │                            ├──│Light emission   │───│Hardware abnormality in   │
                          │                            │  │measuring system │   │measuring system          │
                          │                            │  └─────────────────┘   └──────────────────────────┘
                          │                            │  ┌─────────────────┐   ┌──────────────────────────┐
                          │                            └──│External          │───│Occurrence of leak of     │
                          │                               │disturbance light│   │external disturbance light│
                          │                               └─────────────────┘   └──────────────────────────┘
                          │  ┌─────────────────────┐   ┌─────────────────┐   ┌──────────────────────────┐
                          ├──│Background light     │───│Light emission   │───│Hardware abnormality in   │
                          │  │measuring results are│   │measuring system │   │measuring system          │
                          │  │less than lower limit│   └─────────────────┘   └──────────────────────────┘
                          │  │of allowable values. │
                          │  └─────────────────────┘
                          │  ┌─────────────────────┐   ┌─────────────────┐   ┌──────────────────────────┐
                          ├──│Standard deviation of│───│Light emission   │───│Hardware abnormality in   │
                          │  │background light     │   │measuring system │   │measuring system          │
                          │  │measurement is more  │   └─────────────────┘   └──────────────────────────┘
                          │  │than upper limit of  │
                          │  │allowable values.    │
                          │  └─────────────────────┘
                          │  ┌─────────────────────┐   ┌─────────────────┐   ┌──────────────────────────┐
                          └──│Standard deviation of│───│Light emission   │───│Hardware abnormality in   │
                             │background light     │   │measuring system │   │measuring system          │
                             │measurement is less  │   └─────────────────┘   └──────────────────────────┘
                             │than lower limit of  │
                             │allowable values.    │
                             └─────────────────────┘
```

EP 2 343 381 A1

# FIG. 7

| | | | |
|---|---|---|---|
| Abnormality in standard ATP measurement. | Light emission measured values are more than allowable upper limit. | Reagent system | Concentration of ATP of reagent is higher than specified value. |
| | | Dispensing and dividing system | Light emission reagent |
| | | | Standard ATP |
| | | Light emission measuring system | Hardware abnormality in measuring system |
| | | Predetermined data | Standard ATP concentration predetermined value |
| | Light emission measured values are lower than allowable upper limit. | Reagent system | Concentration of ATP of reagent is lower than specified value. |
| | | Dispensing and dividing system | Light emission reagent |
| | | | Standard ATP |
| | | Light emission measuring system | Hardware abnormality in measuring system |
| | | Predetermined data | Standard ATP concentration predetermined value |
| | Attenuation coefficient is out of allowable range. | Reaction condition | Temperature condition is out of specified range. |
| | | | Occurrence of external disturbance light during measurement of light emission. |
| | | | Occurrence of vibration during measurement of light emission. |
| | Abrupt occurrences of data large in difference from attenuation curve. | Reaction condition | Occurrence of vibration during measurement of light emission. |
| | | | Occurrence of external disturbance light during measurement of light emission. |
| | | Light emission measuring system | On/off of power source of PMT during measurement. |

EP 2 343 381 A1

# F I G. 8

| Abnormality in sample measurement. | Background noise level difference between standard ATP and sample measurement is more than allowable values. | Reagent system | Occurrence of ATP contamination in reagent rack. |
|---|---|---|---|
| | | Dispensing and dividing system | Dispensing and dividing amount is not stable. |
| | | Light emission measuring system | ATP in standard ATP measurement is not eliminated completely. |
| | | | Occurrence of leak of external disturbance light. |
| | No significant difference between counts of light emission measurement and counts of background noise. | Sample | Measurement in aseptic state (normal case). |
| | | | Leakage occurs in filtering process, and bacteria cannot be dispensed in the liquid system. |
| | | Dispensing and dividing system | The reagent is spent up in the process before the previous step, and the reagent cannot be dispensed in the light emission measurement. |
| | Measured values in light emission measurement exceed allowable values. | Sample | ATP more than allowable value is contained. |
| | Difference in attenuation rate of light emission difference between standard ATP measurement and sample measurement is more than allowable values. | Dispensing and dividing system | Abnormality of nozzle discharge speed. |
| | | Light emission measuring system | External disturbance light is generated during light emission measurement. |
| | | | Vibration is generated during light emission measurement. |

# FIG. 9

```
                    ┌──────────────┐
                    │    Start     │
                    └──────┬───────┘
                           │
                           ▼
              ┌─────────────────────────┐
              │  Calculation of judging │
              │ conditions in Rules 1 to 8. │
              └────────────┬────────────┘
                           │
                           ▼
                   ╱─────────────╲        One or more items being out of allowable range.
                  ╱  (1) to (7) being ╲───────────────────────────────────────────┐
                  ╲  within allowable ╱                                            │
                   ╲     range?     ╱                                              │
                    ╲─────┬───────╱                                               │
                          │ All within allowable range.                          │
                          ▼                                                       │
                   ╱─────────────╲      There is significant difference.          │
                  ╱ No significant ╲──────────────────────────┐                   │
                  ╲  difference in  ╱                          │                   ▼
                   ╲     (8)?      ╱                           │          ┌──────────────────────┐
                    ╲─────┬───────╱                           │          │ Display of measured values. │
                          │ No significant difference.        │          └───────────┬──────────┘
                          ▼                                    │                      │
              ┌────────────────────┐                          │                      ▼
              │ Inability of measurement is │            ┌──────────────────┐  ┌──────────────────────┐
              │      displayed.      │            │ Display of measured │  │  Simultaneous listing of │
              └──────────┬──────────┘            │      values.      │  │    judging results.    │
                         │                        └─────────┬────────┘  └───────────┬──────────┘
                         ▼                                   │                       │
                   ╱─────────────╲   Leak is present.        │                       │
                  ╱  No leakage in ╲──────────┐              │                       │
                  ╲    filtering   ╱          │              │                       │
                   ╲   process?   ╱           │              │                       │
                    ╲─────┬───────╱           │              │                       ▼
                          │ No leak.          │              │          ┌──────────────────────┐
                          ▼                    ▼              │          │  Checking or repair is  │
              ┌────────────────────┐  ┌──────────────┐       │          │      required.        │
              │ Measured results are regarded │ │ Repair required. │      │          └───────────┬──────────┘
              │ normal (aseptic being lower │ └───────┬──────┘       │                       │
              │ than measurement limits). │         │               │                       │
              └──────────┬──────────┘         │               │                       │
                         │                     │               │                       │
                         ├─────────────────────┴───────────────┴───────────────────────┘
                         ▼
                    ┌──────────────┐
                    │     End      │
                    └──────────────┘
```

EP 2 343 381 A1

# F I G. 1 O (a)

( a )

Allowable range when calculated
by attenuation rate alone

Region DD

When coming closer to the
intensity of the background light,
it is easy to be departed from
the allowable values,
and it is required to permit
a larger allowable value.

# F I G. 1 O (b)

Region DD

Allowable range when corrected
by using C4 as asymptotic line.

Europäisches
Patentamt

European
Patent Office

Office européen
des brevets

## EUROPEAN SEARCH REPORT

Application Number

EP 11 15 0476

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2009/142785 A1 (OSATO NOE [JP] ET AL) 4 June 2009 (2009-06-04) * the whole document * ----- | 1-10 | INV. C12Q1/04 C12Q1/66 C12M1/34 |
| A | US 5 773 710 A (SQUIRRELL DAVID JAMES [GB]) 30 June 1998 (1998-06-30) * the whole document * ----- | 1-10 | |
| A | US 3 797 999 A (WITZ S ET AL) 19 March 1974 (1974-03-19) * the whole document * ----- | 1-10 | |

TECHNICAL FIELDS
SEARCHED      (IPC)

C12Q
C12M

The present search report has been drawn up for all claims

| Place of search | Date of completion of the search | Examiner |
|---|---|---|
| The Hague | 4 March 2011 | Jenkins, Gareth |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.......................................................................................
& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 11 15 0476

This annex lists the patent family members relating to the patent documents cited in the above-mentioned European search report.
The members are as contained in the European Patent Office EDP file on
The European Patent Office is in no way liable for these particulars which are merely given for the purpose of information.

04-03-2011

| Patent document cited in search report | | Publication date | Patent family member(s) | | Publication date |
|---|---|---|---|---|---|
| US 2009142785 | A1 | 04-06-2009 | SG | 153027 A1 | 29-06-2009 |
| US 5773710 | A | 30-06-1998 | AT | 201715 T | 15-06-2001 |
| | | | AU | 699575 B2 | 10-12-1998 |
| | | | AU | 1858895 A | 09-10-1995 |
| | | | CA | 2195956 A1 | 28-09-1995 |
| | | | DE | 69521140 D1 | 05-07-2001 |
| | | | DE | 69521140 T2 | 11-10-2001 |
| | | | DK | 789778 T3 | 30-07-2001 |
| | | | EP | 0789778 A1 | 20-08-1997 |
| | | | ES | 2156937 T3 | 01-08-2001 |
| | | | WO | 9525811 A1 | 28-09-1995 |
| | | | GR | 3035961 T3 | 31-08-2001 |
| | | | PT | 789778 E | 30-11-2001 |
| | | | RU | 2142016 C1 | 27-11-1999 |
| US 3797999 | A | 19-03-1974 | DE | 2125126 A1 | 02-12-1971 |
| | | | GB | 1315467 A | 02-05-1973 |
| | | | JP | 56011892 B | 17-03-1981 |

EPO FORM P0459

For more details about this annex : see Official Journal of the European Patent Office, No. 12/82

**EP 2 343 381 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009139115 A